# EUROPEAN PATENT SPECIFICATION

(11) **EP 4 511 838 B1**
(45) Date of publication and mention of the grant of the patent: **28.10.2026**
(21) Application number: 23755185.8
(22) Date of filing: 28.06.2023
(51) Int. Cl.: G16B 40/20, G16B 20/00, C12Q 1/6886

(54) **METHOD AND SYSTEM FOR DETECTING TUMOUR PRESENCE FROM MAPPING METRICS OF FREE CIRCULATING DNA FRAGMENTS**
VERFAHREN UND SYSTEM ZUM NACHWEIS VON TUMORPRÄSENZ AUS KARTIERUNGSMETRIKEN VON FREI ZIRKULIERENDEN DNA-FRAGMENTEN
PROCÉDÉ ET SYSTÈME DE DÉTECTION DE PRÉSENCE DE TUMEUR À PARTIR DE MESURES DE MAPPAGE DE FRAGMENTS D'ADN LIBRE CIRCULANT

(43) Date of publication of application: 26.02.2025
(73) Proprietor: GENETON s.r.o., 841 04 Bratislava (SK)
(72) Inventor: KRAMPL, Werner, 831 06 Bratislava (SK); BUDIS, Jaroslav, 851 02 Bratislava (SK); SZEMES, Tomas, 841 03 Bratislava (SK)
(74) Representative: inventa Patentova a znamkova kancelaria s.r.o.
(86) International application number: PCT/SK2023/050021
(87) International publication number: WO 2025/005892

(56) References cited:
- WO-A1-2022/203437
- WO-A1-2023/281111
- US-A1- 2022 136 062
- BRUEFFER CHRISTIAN ET AL: "Quality Control and Analysis of RNA-seq Data from Breast Cancer Tumor Samples", 31 December 2013 (2013-12-31), pages 1 - 20, XP093118157, Retrieved from the Internet <URL:http://lup.lub.lu.se/student-papers/record/3920650/file/3920651.pdf> [retrieved on 20240111]
- FLORENT MOULIERE ET AL: "Enhanced detection of circulating tumor DNA by fragment size analysis", SCIENCE TRANSLATIONAL MEDICINE, vol. 10, no. 466, 7 November 2018 (2018-11-07), pages eaat4921, XP055669959, ISSN: 1946-6234, DOI: 10.1126/scitranslmed.aat4921

## Description

### Field of the invention

The invention generally relates to DNA diagnostics and bioinformatics and specifically deals with the detection of the presence of a tumor from free circulating DNA. The invention belongs to the field of computational biology and biotechnology.

### Prior art

Prior knowledge of machine learning cancer prediction based on sequenced free circulating DNA (cfDNA) is published in numerous scientific studies and patents. Starting with the seminal work of Mandel and Metaisaz in 1948, which first describes circulating DNA (Mandel P, Metais P. Les acidesnucléiquesduplasmasanguinchezl'homme. C R SeancesSocBiolFil 1948; 142(3-4): 241-3). Another important milestone in the research of circulating DNA in 1989, the presence of the so-called circulating free fetal DNA (cffDNA-cellfreefetal DNA) (Lo YM, Patel P, Wainscoat JS, et al. Prenatal sex determination by DNA amplification from maternal peripheral blood. Lancet 1989; 2(8676): 1363-5). In parallel with research in the field of cffDNA analysis, it was possible to follow similar research in the field of circulating tumor DNA analysis (ctDNA-circulating tumor DNA). Fast forward to 2013 when Dawson et al. performed a landmark study that involved sequencing ctDNA from plasma samples of patients with metastatic breast cancer, paving the way for the use of ctDNA as a biomarker for cancer detection and monitoring.

At the same time, the application of machine learning techniques in cancer prediction and prognosis has begun to gain traction, as documented in detail in a systematic review by Kourou et al. in 2015. Different types of input data, including clinical, genomic, proteomic and imaging data, were considered in these machine learning models.

Later, the union of these two fields, ectDNA sequencing and machine learning, materialized in a pioneering study by Wan et al. in 2017. They innovatively applied machine learning methods to cancer prediction, using array-based comparative genomichybridization (aCGH) data and ctDNA-derived gene expression profiling. This pioneering work set the precedent for subsequent research efforts in this area.

Based on this, Phallen et al. in 2018, they introduced a deep learning method, CancerSEEK, which directly detected early-stage cancer using ctDNA sequencing data, protein biomarker levels, and clinical data. Their work focused on mutations in 16 genes that are frequently altered in different types of cancer, as well as eight protein biomarkers associated with cancer.

Moreover, Liu et al. in 2019 used genomic data from genome-wide association studies (GWAS), specifically single nucleotide polymorphisms (SNPs), to build breast cancer risk prediction models using machine learning algorithms, confirming the potential versatility and utility of such models.

These scientific breakthroughs are complemented by a series of patents that offer valuable insights into the application of machine learning in genomic medicine. Google LLC, in their patent US20170342540A1, applied deep learning algorithms to digital pathology images to predict lymph node metastases in cancer patients. Similarly, AppliedProteomics, Inc., in its patent US9741057B2, demonstrated the use of machine learning in genomic medicine to predict drug responses using proteomic and genomic data.

The international application WO2023281111A1 introduces a method to analyze urine samples for signs of brain cancer by examining tiny pieces of DNA that are released into the urine. By measuring the size of these DNA fragments and using advanced computer algorithms (machine learning), the method can predict whether a sample is likely from a brain cancer patient or not.

The international application WO2022203437A1 discloses a method for detecting cancer early by analysing tiny fragments of DNA found in the blood, known as cell-free DNA. It uses artificial intelligence to identify specific mutations that indicate the presence of tumors, making the diagnosis more accurate and sensitive.

A scientific article by Brueffer Christian ET AL: "Quality Control and Analysis of RNA-seq Data from Breast Cancer Tumor Samples", published 31 December 2013, discusses the development and integration of an RNA-seq quality control pipeline into the SCAN-B RNA-seq analysis pipeline, which was used to evaluate the quality of 2547 RNA-seq libraries from breast cancer tumor samples, showing overall good data quality with improvements over time. The document highlights the importance of quality control in RNA-seq analysis, especially given the distinct genomic landscape of cancer cells, which can complicate the interpretation of quality metrics.

### Background of the invention

### Definitions

In the context of the present invention, the term "genome" refers to the complete set of DNA sequences in an organism.

"ctDNA" (circulatingtumour DNA) is a type of extracellular free DNA found in the peripheral blood of patients with oncological disease. DNA fragments are released into the circulation after apoptosis and necrosis of cells, and their amount correlates with the stage of the disease and the prognosis. In addition, determination of the genotype of tumor cells makes it possible to detect and quantify tumor mutations in real time.

As used herein, the term "variant/variation" refers to a difference between a genome and a reference genome.

The term "reference genome" as used herein refers to a representative example of the genome of a species to which sequencing reads map.

The term "DNA sequencing" refers to techniques enabling the precise determination of the sequence of nucleic base pairs in an organism.

As used herein, the term "read" refers to the deduced sequence of base pairs (or base pair probabilities) corresponding to all or part of a single DNA fragment. In other words, they are small contiguous parts of an individual's DNA. The read should be long enough to serve as a sequence tag so that it can be unambiguously mapped or assigned to an exact location in the reference genome - at least 30-35 bp.

The term "mapping" refers to the alignment of sequence information from NGS (i.e., a DNA fragment whose genomic position is unknown) to the corresponding sequence in the reference human genome. This alignment can be done in several ways. Readers that do not map unambiguously (map to several positions) are usually excluded from the analysis. Alignment is typically performed using computer algorithms well known to those skilled in the art of molecular biology and bioinformatics.

The term "VCF file" refers to a file that contains the variants of an individual in a concise format. The VCF format is also known to bioinformatics experts as the standard format for storing variants of an individual.

The term "annotation" refers to the process of identifying the location of genes and other coding regions in the genome, as well as other sites of interest. Annotation can also provide additional information (e.g. purpose of genes, etc.).

The term "FASTQ file" in this document means a file containing all reads from a sequencer along with their sequencing quality. This is a standard file format for storing this data, which is usually compressed to save disk space. All modern mapping software accepts this format as input.

The term "SAM/BAM file" in this document refers to a file that contains aligned sequence reads in text format (SAM) or compressed binary format (BAM).

For each read, it contains its mapped position on the reference genome (if mapping for that read was successful), mapping quality, sequencing quality (if provided), paired read location (if paired sequencing), and various other information. It is a standard for storing aligned reads. Each SAM/BAM file depends on the reference genome used - this information is stored in the header of the SAM/BAM file.

"PCR - polymerase chain reaction (PCR)" is a molecular technique that makes it possible to create millions of copies of a short stretch of DNA through repeated cycles of denaturation, annealing and elongation.

The first step is the collection and processing of biological samples (e.g. blood plasma, saliva, urine, etc.) obtained from healthy persons and cancer patients. First, each sample needs to be biochemically prepared for the sequencing process, which usually involves the following steps.

DNA is isolated from a biological sample using biochemical and physical techniques (the exact technique depends on the origin of the sample). The DNA is then further processed to a state suitable for sequencing (or any other method used to obtain digital information about the base order and other properties of the processed DNA), usually a sequencing library. The processed DNA sample is subjected to massively parallel sequencing by NGS approach.

After the sequencing step, the organism's genome is obtained in digital form in the form of sequencing reads (usually a FASTQ file). Sequencing reads are then mapped to a reference genome (typically creating a SAM/BAM file).

The mapped readings are subsequently statistically processed, while statistical metrics such as e.g. (but not limited to) the number of mapped reads, the number of unmapped reads, the length of DNA fragments and so on.

These statistics are subsequently processed by statistical procedures or machine learning procedures, which determine the samples with the predicted occurrence of the tumor. Primarily, but not exclusively, the procedure involves anomaly detection, where we consider the tumor sample to be an anomaly. The mapped reads from the samples are then divided into a training and a test set. To detect the anomaly, a machine learning model is trained using the training set, while the said model classifies the samples as healthy and tumorous. The detection accuracy is subsequently validated on the test set.

A new, unknown sample is subsequently determined by the same biochemical and bioinformatics procedure. Subsequently, its condition is evaluated using the trained and validated model described above.

The above-described methods of the invention can be implemented in the form of modules and sub-modules in a computer system that includes computing device(s), server(s) and means for mutual data communication (e.g. LAN, Internet) and for data communication with another ( -i) computer system(s) and databases, either implemented as part of the computer system itself or as an external server.

Computing devices and servers may include a processor (central processing unit, CPU), a graphics processor (graphics processing unit, GPU), random access memory (RAM), nonvolatile secondary storage such as a hard disk, network interfaces, and peripherals, including means for interface with the user such as keyboard and display. Program code, including software programs, and data are loaded into RAM for execution and processing by the processor, and results are generated for display, output, transmission, or storage.

Modules and submodules configured to perform one or more steps of the invention may be implemented as a computer program or procedure written as source code in a common programming language and submitted for execution to a CPU or GPU as object or byte code. Alternatively, the modules and sub-modules can also be implemented in hardware, either as integrated circuits or burned into read-only memory components, and then each of the computing devices and the server can function as a dedicated computer. Various implementations of source code and object and byte codes can be stored on a computer-readable storage medium such as a hard disk drive (HDD), solid state disk (SSD), flash disk, random access memory (RAM), read-only memory (ROM) and similar storage media.

Other types of modules and module functions, as well as other physical hardware components, are possible as known to those skilled in the art.

A computer system configured to process anomalous samples includes modules configured to perform sequencing read processing, variant calling, MSI status analysis, model training and testing, and classification of new samples.

Another object of this invention is a computer program product containing computer-readable instructions which, when loaded and executed in a computer system, cause the computer system to perform operations according to the method of the invention.

A typical computer system is configured as follows: an analytical computer system consists of either a single system that performs all the calculations, or it is a computer server that distributes the calculations to several computing nodes. Each computing node then performs part or all of the required set of calculations and delivers the results of the calculations back to the computer server.

The mentioned invention and system differs from the current state of the art based on the input data, which in this case are mapping statistics, which requires a minimum of information compared to other procedures used to detect the presence of a tumor in a sample. For the above reasons, since it is not necessary to obtain additional information, sample processing is faster and saves costs associated with the operation of a computer system designed to detect the presence of a tumor compared to other methods.

### Examples of embodiments of the invention

### Example 1

All readings from the dataset of colorectal cancer patients and healthy controls are analyzed using next-generation sequencing (NGS) technology, namely Illumina's sequencing platform with the Truseq sequencing kit with 100 bp reads, yielding datasets in FASTQ format.

The sequencing quality of individual samples is subsequently verified by the FastQC tool designed for sequencing quality control. The samples are subsequently modified using Trimmomatic tools, or TrimGalore, which allows to remove sequencing adapters or other artifacts from the reads, to remove those reads that, based on PhredScore, do not have the required quality (typically an average PhredScore of 20 for the entire read) or are too short (typically less than 75 bp).

The resulting number of samples after adjustments is subsequently mapped to the reference human genome GRCh38.p12, or another suitable version of the genome, using BWA-MEM or Bowtie2. Mapped reads are saved in SAM format. Subsequently, the processes of compression, sorting of mapped reads and their deduplication will take place, during which the reads that are repeated for the given sample (have been sequenced several times) and which are not continued in further analyzes are marked. The result is a BAM file, i.e. a binary SAM file, which is a compressed version of it. These steps are done using Samtools.

After these steps, 153 mapped samples are finally available, of which 126 are control and 27 are colorectal cancers. Subsequently, the mapping statistics are calculated using the Qualimap tool and using a custom script in the Python3 programming language.

The statistics used include, but are not limited to: the number of sequenced reads, the number of mapped reads to the reference genome, the ratio of mapped reads to the reference genome to all reads, the number of reads with different positions on the genome within a single read, the ratio of all reads with different positions on the genome in within one read to all reads, number of reads with two or more positions on the genome, ratio of all reads with two or more positions on the genome, number of pairs of reads with the first read of the pair mapped, number of pairs of reads with the second read of the pair mapped, number of pairs of reads with both reads from a pair mapped, number of pairs of reads with only one read from a pair mapped, number of bases sequenced, number of bases mapped, number of labeled duplicate reads, average DNA fragment length, standard deviation of DNA fragment lengths, weighted average of DNA fragment lengths, median length of DNA fragments, weighted median length of DNA fragments, average mapping quality, median mapping quality, number of adenine bases in mapped reads, ratio of adenine bases to all bases, number of cytosine bases in mapped reads, ratio of cytosine bases to all bases, number of thymine bases in of mapped reads, ratio of thymine bases to all bases, number of guanine bases in mapped reads, ratio of guanine bases to all bases, number of unknown bases in mapped reads, ratio of unknown bases to all bases, ratio of base mismatches to all mapped bases, number of substituted bases against references, number of inserted bases against references, number of deletions against references, number of reads with insertion, number of reads with deletion, number of homopolymeric insertions and deletions, average depth of sequencing coverage, standard deviation of depths of sequencing coverage, median of depths of sequencing coverage, number of bases mapped to individual chromosomes, the average depth of sequencing coverage of individual chromosomes, the standard deviation of the average depth of sequencing coverage of individual chromosomes, the number of DNA fragments with lengths from the minimum sequenced length to the maximum sequenced length separately for each length, the ratio of DNA fragments with lengths from the minimum sequenced length to the maximum sequenced length separately for each length against all sequenced DNA fragments and other statistics.

Selected statistics (for the purpose of describing the invention) for two samples can be seen in Table 1. These statistics are subsequently extracted and stored in Flat JSON format using a custom Python3 script.

The samples are subsequently divided into a training and a test set (Tab. 2). There are 101 control samples and 21 patient samples in the training set. There are 25 control samples and 6 patient samples in the test set.

Subsequently, a machine learning model of the Anomaly Detection category is trained. The Extreme Gradient BoostingforOutlierDetection (XGBOD) model is chosen as the prediction model, but the model can be any machine learning model. After training the model with the training set described above, the model is tested with the test set, and the prediction testing results are described in Table 3.

**Table 1 Example of selected statistics for two samples**

| | **lycc_crc_0n7dj_01_01_pl** | **lycc_crc_0n7dj_02_01_pl** |
|---|---|---|
| **Number of reads sequenced** | 45142402 | 45341520 |
| **Number of reads mapped** | 45033044 | 45242760 |
| **Ratio of mapped reads to the reference genome to all reads** | 0.9976 | 0.9978 |
| **Number of reads with different positions on the genome within a single read** | 73662 | 73434 |
| **The ratio of all reads with different positions on the genome within one read to all reads** | 0.0016 | 0.0016 |
| **Number of reads with two or more positions on the genome** | 0 | 0 |
| **Number of pairs of reads with the first read of the pair mapped** | 22517002 | 22621955 |
| **Number of pairs of reads with the second read from the pair mapped** | 22516042 | 22620805 |
| **Number of read pairs with both reads from the pair mapped** | 45026002 | 45235364 |
| **Number of pairs of reads with only one read from the pair mapped** | 7042 | 7396 |
| **Number of bases mapped** | 4.43E+09 | 4.45E+09 |
| **Number of bases sequenced** | 4.43E+09 | 4.45E+09 |

**Table 2 Division of control and patient samples into training and testing sets.**

| | **Training set** | **Testing set** | **Sum** |
|---|---|---|---|
| **Control samples** | 101 | 25 | 126 |
| **Patient samples** | 21 | 6 | 27 |
| **Sum** | 122 | 31 | 153 |

**Table 3 Results of testing the XGBOD model on the test set. Individual metrics are defined by their standard meaning in the field of machine learning.**

| | |
|---|---|
| True negative | 25 |
| True positive | 3 |
| False negative | 3 |
| False positive | 0 |
| Accuracy | 1,0 |
| Coverage | 0,5 |
| Correctness | 0,90 |
| F1 | 0,67 |

## Claims

1. A computer-implemented method for determining the presence of a tumour from mapping metrics of free circulating DNA fragments in a test sample, **characterized in that** it comprises the following steps:
a. creating a training data set by calculating statistical metrics over a mapped sequencing data from a training samples containing a number of mapped reads, a number of unmapped reads, a length of DNA fragments and information about the presence of a tumour;
b. training a statistical model or a machine learning model using the tumour presence information from step a) as model output and other statistical metrics from step a) as model input,
c. providing a tested sample and calculating statistical metrics over its sequencing data, including the number of mapped reads, the number of unmapped reads and the length of DNA fragments,
d. predicting the presence of a tumour in the test sample using the trained statistical model or machine learning model from step b) and statistical metrics from step c) as its input.

2. The method according to claim 1, where the statistical metrics calculated over the mapped sequencing data from the training samples in step a) further contain any data from the set containing: the number of sequenced reads, the ratio of mapped reads to the reference genome to all reads, the number of reads with different positions on the genome within one read, the ratio of all reads with different positions on the genome within one read to all reads, the number of reads with two or more positions on the genome, the ratio of all reads with two or more positions on the genome, the number of paired reads with the first read mapped from of a pair, number of read pairs with the second read from a pair mapped, number of read pairs with both reads from a pair mapped, number of read pairs with only one read from a pair mapped, number of bases sequenced, number of bases mapped, number of labelled duplicate reads, average mapping quality, median mapping quality, number of adenine bases in mapped reads, ratio of adenine bases to all bases, number of cytosine bases in mapped reads, ratio of cytosine bases to all bases, number of thymine bases in mapped reads, ratio of thymine bases to all bases, number of guanine bases in of mapped reads, ratio of guanine bases to all bases, number of unknown bases in mapped reads, ratio of unknown bases to all bases, ratio of base mismatches to all mapped bases, number of substituted bases to references, number of inserted bases to references, number of deletions to references, number of reads with insertion, number of reads with deletion, number of homopolymeric insertions and deletions, average depth of sequencing coverage, standard deviation of depths of sequencing coverage, median of depths of sequencing coverage, number of bases mapped to individual chromosomes, average depth of sequencing coverage of chromosomes, standard deviation of averages of individual depths sequencing coverage of individual chromosomes.

3. The method according to claim 2, where the statistical metrics calculated over the mapped sequencing data from the tested sample in step c) contain the same statistical metrics as were used to create the training data set.

4. The method according to any one of the preceding claims, wherein the statistics are derived from incomplete genomic data.

5. The statistical model or model for the machine determination of the presence of a tumour from the mapping metrics of fragments of free circulating DNA in a test sample used in the method according to any of the preceding claims containing at the input statistical metrics from the mapped sequencing data of the sample providing for the number of mapped reads, the number of unmapped reads, the length of DNA fragments at the input, information about the presence of a tumour at the output.

6. The model according to claim 5, further containing at the input any data from the set containing: the number of sequenced reads, the ratio of mapped reads to the reference genome to all reads, the number of reads with different positions on the genome within one read, the ratio of all reads with different positions on genomes within one read versus all reads, number of reads with two or more positions on the genome, ratio of all reads with two or more positions on the genome, number of pairs of reads with the first read of the pair mapped, number of pairs of reads with the second read of the pair mapped, number of read pairs with both reads from a pair mapped, number of read pairs with only one read from a pair mapped, number of bases sequenced, number of mapped bases, number of tagged duplicate reads, average mapping quality, median mapping quality, number of adenine bases in mapped reads, ratio of adenine bases to all bases, number of cytosine bases in mapped reads, ratio of cytosine bases to all bases, number of thymine bases in mapped reads, ratio of thymine bases to all bases, number of guanine bases in mapped reads, ratio of guanine bases to all bases, number of unknown bases in mapped reads, ratio of unknown bases to all bases, ratio of base mismatches to all mapped bases, number of substituted bases to references, number of inserted bases to references, number of deletions to references, number of reads with insertions, number of reads with deletions, number of homopolymers of insertions and deletions, average depth of sequencing coverage, standard deviation of depths of sequencing coverage, median depth of sequencing coverage, number of bases mapped to individual chromosomes, average depth of sequencing coverage of individual chromosomes, standard deviation of average depths of sequencing coverage of individual chromosomes.

7. The computer-implemented method of creating a training set of data for a statistical model or machine determination model according to claim 5 comprising the following steps:
a. creating a training data set by calculating statistical metrics over the mapped sequencing data from the training samples containing the number of mapped reads, the number of unmapped reads, the length of DNA fragments and information about the presence of a tumour,
b. training a statistical model or a machine learning model using the tumour presence information from step a) as model output and other statistical metrics from step a) as model input.

8. The method according to claim 7, where the statistical metrics calculated over the mapped sequencing data from the training samples in step a) further contain any data from the set containing: the number of sequenced reads, the ratio of mapped reads to the reference genome to all reads, the number of reads with different positions on genomes within a single read, the ratio of all reads with different positions on the genome within a single read to all reads, the number of reads with two or more positions on the genome, the ratio of all reads with two or more positions on the genome, the number of paired reads with the first read mapped of a pair, number of read pairs with second read of a pair mapped, number of read pairs with both reads of a pair mapped, number of read pairs with only one read of a pair mapped, number of bases sequenced, number of bases mapped, number of duplicate reads tagged, average mapping quality , median mapping quality, number of adenine bases in mapped reads, ratio of adenine bases to all bases, number of cytosine bases in mapped reads, ratio of cytosine bases to all bases, number of thymine bases in mapped reads, ratio of thymine bases to all bases, number of guanine bases in mapped reads, ratio of guanine bases to all bases, number of unknown bases in mapped reads, ratio of unknown bases to all bases, ratio of base mismatches to all mapped bases, number of substituted bases to references, number of inserted bases to references, number of deletions to references , number of reads with insertion, number of reads with deletion, number of homopolymer insertions and deletions, average depth of sequencing coverage, standard deviation of depths of sequencing coverage, median of depths of sequencing coverage, number of bases mapped to individual chromosomes, average depth of sequencing coverage of individual chromosomes, standard deviation of means depth of sequencing coverage of individual chromosomes.

9. A computer system comprising computing devices configured to perform the method of any one of claims 1 to 4, 7 or 8.

10. A computer program containing instructions which, if executed by a computer, ensure the implementation of the method according to any one of claims 1 to 4, 7 or 8 by this computer.

11. A computer data medium containing program instructions which, when executed by a computer, ensure the implementation of the method according to any one of claims 1 to 4, 7 or 8 by this computer.

## Patentansprüche

1. Computerimplementiertes Verfahren zur Bestimmung des Vorliegens eines Tumors anhand von Kartierungsmetriken von Fragmenten zellfreier zirkulierender DNA in einer Testprobe, **dadurch gekennzeichnet, dass** es die folgenden Schritte umfasst:
a. Erstellen eines Trainingsdatensatzes durch Berechnung statistischer Metriken aus kartierten Sequenzierungsdaten von Trainingsproben, wobei die statistischen Metriken die Anzahl der kartierten Reads, die Anzahl der nicht kartierten Reads, die Länge von DNA-Fragmenten und Informationen über das Vorliegen eines Tumors umfassen;
b. Trainieren eines statistischen Modells oder eines Modells des maschinellen Lernens unter Verwendung der Information über das Vorliegen eines Tumors aus Schritt a) als Ausgabe des Modells und der übrigen statistischen Metriken aus Schritt a) als Eingabe des Modells;
c. Bereitstellen einer Testprobe und Berechnen statistischer Metriken aus deren Sequenzierungsdaten, wobei die statistischen Metriken die Anzahl der kartierten Reads, die Anzahl der nicht kartierten Reads und die Länge von DNA-Fragmenten umfassen;
d. Vorhersagen des Vorliegens eines Tumors in der Testprobe unter Verwendung des in Schritt b) trainierten statistischen Modells oder Modells des maschinellen Lernens und der statistischen Metriken aus Schritt c) als dessen Eingabe.

2. Verfahren nach Anspruch 1, wobei die in Schritt a) aus den kartierten Sequenzierungsdaten der Trainingsproben berechneten statistischen Metriken ferner beliebige Daten aus der Gruppe umfassen, die Folgendes umfasst: die Anzahl der sequenzierten Reads, ein Verhältnis der auf das Referenzgenom kartierten Reads zu allen Reads, die Anzahl der Reads mit unterschiedlichen Positionen auf dem Genom innerhalb eines einzelnen Reads, ein Verhältnis aller Reads mit unterschiedlichen Positionen auf dem Genom innerhalb eines einzelnen Reads zu allen Reads, die Anzahl der Reads mit zwei oder mehr Positionen auf dem Genom, ein Verhältnis aller Reads mit zwei oder mehr Positionen auf dem Genom, die Anzahl der Read-Paare mit einem kartierten ersten Read des Paares, die Anzahl der Read-Paare mit einem kartierten zweiten Read des Paares, die Anzahl der Read-Paare mit beiden kartierten Reads des Paares, die Anzahl der Read-Paare mit nur einem kartierten Read des Paares, die Anzahl der sequenzierten Basen, die Anzahl der kartierten Basen, die Anzahl der als dupliziert markierten Reads, die durchschnittliche Mapping-Qualität, der Median der Mapping-Qualität, die Anzahl der Adeninbasen in den kartierten Reads, ein Verhältnis der Adeninbasen zu allen Basen, die Anzahl der Cytosinbasen in den kartierten Reads, ein Verhältnis der Cytosinbasen zu allen Basen, die Anzahl der Thyminbasen in den kartierten Reads, ein Verhältnis der Thyminbasen zu allen Basen, die Anzahl der Guaninbasen in den kartierten Reads, ein Verhältnis der Guaninbasen zu allen Basen, die Anzahl der unbekannten Basen in den kartierten Reads, ein Verhältnis der unbekannten Basen zu allen Basen, ein Verhältnis der Basenfehlpaarungen zu allen kartierten Basen, die Anzahl der gegenüber der Referenz substituierten Basen, die Anzahl der gegenüber der Referenz inserierten Basen, die Anzahl der gegenüber der Referenz deletierten Basen, die Anzahl der Reads mit einer Insertion, die Anzahl der Reads mit einer Deletion, die Anzahl der Homopolymer-Insertionen und -Deletionen, die durchschnittliche Sequenzabdeckungstiefe, die Standardabweichung der Sequenzabdeckungstiefen, der Median der Sequenzabdeckungstiefen, die Anzahl der auf die einzelnen Chromosomen kartierten Basen, die durchschnittliche Sequenzabdeckungstiefe der einzelnen Chromosomen und die Standardabweichung der durchschnittlichen Sequenzabdeckungstiefen der einzelnen Chromosomen.

3. Verfahren nach Anspruch 2, wobei die in Schritt c) aus den kartierten Sequenzierungsdaten der Testprobe berechneten statistischen Metriken dieselben statistischen Metriken umfassen, die bei der Erstellung des Trainingsdatensatzes verwendet wurden.

4. Verfahren nach einem der vorhergehenden Ansprüche, wobei die statistischen Metriken aus unvollständigen genomischen Daten stammen.

5. Statistisches Modell oder Modell des maschinellen Lernens zur Bestimmung des Vorliegens eines Tumors anhand von Kartierungsmetriken von Fragmenten zellfreier zirkulierender DNA in einer Testprobe, das in dem Verfahren nach einem der vorhergehenden Ansprüche verwendet wird, wobei das statistische Modell oder das Modell des maschinellen Lernens als Eingabe statistische Metriken aus kartierten Sequenzierungsdaten der Probe umfasst und für die Anzahl der kartierten Reads, die Anzahl der nicht kartierten Reads und die Länge von DNA-Fragmenten als Eingabe sowie Informationen über das Vorliegen eines Tumors als Ausgabe ausgelegt ist.

6. Modell nach Anspruch 5, ferner umfassend als Eingabe beliebige Daten aus der Gruppe, die Folgendes umfasst: die Anzahl der sequenzierten Reads, ein Verhältnis der auf das Referenzgenom kartierten Reads zu allen Reads, die Anzahl der Reads mit unterschiedlichen Positionen auf dem Genom innerhalb eines einzelnen Reads, ein Verhältnis aller Reads mit unterschiedlichen Positionen auf dem Genom innerhalb eines einzelnen Reads zu allen Reads, die Anzahl der Reads mit zwei oder mehr Positionen auf dem Genom, ein Verhältnis aller Reads mit zwei oder mehr Positionen auf dem Genom, die Anzahl der Read-Paare mit einem kartierten ersten Read des Paares, die Anzahl der Read-Paare mit einem kartierten zweiten Read des Paares, die Anzahl der Read-Paare mit beiden kartierten Reads des Paares, die Anzahl der Read-Paare mit nur einem kartierten Read des Paares, die Anzahl der sequenzierten Basen, die Anzahl der kartierten Basen, die Anzahl der als dupliziert markierten Reads, die durchschnittliche Mapping-Qualität, der Median der Mapping-Qualität, die Anzahl der Adeninbasen in den kartierten Reads, ein Verhältnis der Adeninbasen zu allen Basen, die Anzahl der Cytosinbasen in den kartierten Reads, ein Verhältnis der Cytosinbasen zu allen Basen, die Anzahl der Thyminbasen in den kartierten Reads, ein Verhältnis der Thyminbasen zu allen Basen, die Anzahl der Guaninbasen in den kartierten Reads, ein Verhältnis der Guaninbasen zu allen Basen, die Anzahl der unbekannten Basen in den kartierten Reads, ein Verhältnis der unbekannten Basen zu allen Basen, ein Verhältnis der Basenfehlpaarungen zu allen kartierten Basen, die Anzahl der gegenüber der Referenz substituierten Basen, die Anzahl der gegenüber der Referenz inserierten Basen, die Anzahl der gegenüber der Referenz deletierten Basen, die Anzahl der Reads mit einer Insertion, die Anzahl der Reads mit einer Deletion, die Anzahl der Homopolymer-Insertionen und -Deletionen, die durchschnittliche Sequenzabdeckungstiefe, die Standardabweichung der Sequenzabdeckungstiefen, der Median der Sequenzabdeckungstiefen, die Anzahl der auf die einzelnen Chromosomen kartierten Basen, die durchschnittliche Sequenzabdeckungstiefe der einzelnen Chromosomen und die Standardabweichung der durchschnittlichen Sequenzabdeckungstiefen der einzelnen Chromosomen.

7. Computerimplementiertes Verfahren zum Erstellen eines Trainingsdatensatzes für ein statistisches Modell oder ein Modell des maschinellen Lernens nach Anspruch 5, umfassend die folgenden Schritte:
a. Erstellen eines Trainingsdatensatzes durch Berechnung statistischer Metriken aus kartierten Sequenzierungsdaten von Trainingsproben, wobei die statistischen Metriken die Anzahl der kartierten Reads, die Anzahl der nicht kartierten Reads, die Länge von DNA-Fragmenten und Informationen über das Vorliegen eines Tumors umfassen;
b. Trainieren eines statistischen Modells oder eines Modells des maschinellen Lernens unter Verwendung der Information über das Vorliegen eines Tumors aus Schritt a) als Ausgabe des Modells und der übrigen statistischen Metriken aus Schritt a) als Eingabe des Modells.

8. Verfahren nach Anspruch 7, wobei die in Schritt a) aus den kartierten Sequenzierungsdaten der Trainingsproben berechneten statistischen Metriken ferner beliebige Daten aus der Gruppe umfassen, die Folgendes umfasst: die Anzahl der sequenzierten Reads, ein Verhältnis der auf das Referenzgenom kartierten Reads zu allen Reads, die Anzahl der Reads mit unterschiedlichen Positionen auf dem Genom innerhalb eines einzelnen Reads, ein Verhältnis aller Reads mit unterschiedlichen Positionen auf dem Genom innerhalb eines einzelnen Reads zu allen Reads, die Anzahl der Reads mit zwei oder mehr Positionen auf dem Genom, ein Verhältnis aller Reads mit zwei oder mehr Positionen auf dem Genom, die Anzahl der Read-Paare mit einem kartierten ersten Read des Paares, die Anzahl der Read-Paare mit einem kartierten zweiten Read des Paares, die Anzahl der Read-Paare mit beiden kartierten Reads des Paares, die Anzahl der Read-Paare mit nur einem kartierten Read des Paares, die Anzahl der sequenzierten Basen, die Anzahl der kartierten Basen, die Anzahl der als dupliziert markierten Reads, die durchschnittliche Mapping-Qualität, der Median der Mapping-Qualität, die Anzahl der Adeninbasen in den kartierten Reads, ein Verhältnis der Adeninbasen zu allen Basen, die Anzahl der Cytosinbasen in den kartierten Reads, ein Verhältnis der Cytosinbasen zu allen Basen, die Anzahl der Thyminbasen in den kartierten Reads, ein Verhältnis der Thyminbasen zu allen Basen, die Anzahl der Guaninbasen in den kartierten Reads, ein Verhältnis der Guaninbasen zu allen Basen, die Anzahl der unbekannten Basen in den kartierten Reads, ein Verhältnis der unbekannten Basen zu allen Basen, ein Verhältnis der Basenfehlpaarungen zu allen kartierten Basen, die Anzahl der gegenüber der Referenz substituierten Basen, die Anzahl der gegenüber der Referenz inserierten Basen, die Anzahl der gegenüber der Referenz deletierten Basen, die Anzahl der Reads mit einer Insertion, die Anzahl der Reads mit einer Deletion, die Anzahl der Homopolymer-Insertionen und -Deletionen, die durchschnittliche Sequenzabdeckungstiefe, die Standardabweichung der Sequenzabdeckungstiefen, der Median der Sequenzabdeckungstiefen, die Anzahl der auf die einzelnen Chromosomen kartierten Basen, die durchschnittliche Sequenzabdeckungstiefe der einzelnen Chromosomen und die Standardabweichung der durchschnittlichen Sequenzabdeckungstiefen der einzelnen Chromosomen.

9. Computersystem, umfassend Recheneinrichtungen, die dazu konfiguriert sind, das Verfahren nach einem der Ansprüche 1 bis 4, 7 oder 8 auszuführen.

10. Computerprogramm, umfassend Anweisungen, die bei Ausführung durch einen Computer bewirken, dass der Computer das Verfahren nach einem der Ansprüche 1 bis 4, 7 oder 8 ausführt.

11. Computerlesbares Datenträgermedium, umfassend Programmanweisungen, die bei Ausführung durch einen Computer bewirken, dass der Computer das Verfahren nach einem der Ansprüche 1 bis 4, 7 oder 8 ausführt.

## Revendications

1. Procédé mis en œuvre par ordinateur pour déterminer la présence d'une tumeur à partir de métriques de cartographie de fragments d'ADN libre circulant dans un échantillon testé**, caractérisé en ce qu'**il comprend les étapes de:
a. Création d'un ensemble de données d'apprentissage par le calcul de métriques statistiques sur des données de séquençage cartographiées provenant d'échantillons d'apprentissage, comprenant le nombre de lectures cartographiées, le nombre de lectures non cartographiées, la longueur des fragments d'ADN et l'information sur la présence d'une tumeur;
b. Entraînement d'un modèle statistique ou d'un modèle d'apprentissage automatique en utilisant l'information sur la présence d'une tumeur de l'étape a) comme sortie du modèle et les autres métriques statistiques de l'étape a) comme entrée du modèle;
c. Fourniture de l'échantillon testé et calcul de métriques statistiques sur ses données de séquençage, comprenant le nombre de lectures cartographiées, le nombre de lectures non cartographiées et la longueur des fragments d'ADN ;
d. Prédiction de la présence d'une tumeur dans l'échantillon testé en utilisant le modèle statistique ou le modèle d'apprentissage automatique entraîné de l'étape b) et les métriques statistiques de l'étape c) comme son entrée.;

2. Procédé selon la revendication 1, dans lequel les métriques statistiques calculées sur les données de séquençage cartographiées provenant des échantillons d'apprentissage à l'étape a) comprennent en outre n'importe quelle donnée parmi l'ensemble contenant: le nombre de lectures séquencées, le ratio des lectures cartographiées sur le génome de référence par rapport à toutes les lectures, le nombre de lectures avec différentes positions sur le génome au sein d'une même lecture, le ratio de toutes les lectures avec différentes positions sur le génome au sein d'une même lecture par rapport à toutes les lectures, le nombre de lectures avec deux positions ou plus sur le génome, le ratio de toutes les lectures avec deux positions ou plus sur le génome, le nombre de paires de lectures avec la première lecture de la paire cartographiée, le nombre de paires de lectures avec la deuxième lecture de la paire cartographiée, le nombre de paires de lectures avec les deux lectures de la paire cartographiées, le nombre de paires de lectures avec une seule lecture de la paire cartographiée, le nombre de bases séquencées, le nombre de bases cartographiées, le nombre de lectures marquées comme dupliquées, la qualité moyenne de cartographie, la médiane de la qualité de cartographie, le nombre de bases adénine dans les lectures cartographiées, le ratio des bases adénine par rapport à toutes les bases, le nombre de bases cytosine dans les lectures cartographiées, le ratio des bases cytosine par rapport à toutes les bases, le nombre de bases thymine dans les lectures cartographiées, le ratio des bases thymine par rapport à toutes les bases, le nombre de bases guanine dans les lectures cartographiées, le ratio des bases guanine par rapport à toutes les bases, le nombre de bases inconnues dans les lectures cartographiées, le ratio des bases inconnues par rapport à toutes les bases, le ratio de mésappariements de bases par rapport à toutes les bases cartographiées, le nombre de bases substituées par rapport à la référence, le nombre de bases insérées par rapport à la référence, le nombre de délétions par rapport à la référence, le nombre de lectures avec insertion, le nombre de lectures avec délétion, le nombre d'insertions et de délétions homopolymériques, la profondeur moyenne de couverture de séquençage, l'écart-type des profondeurs de couverture de séquençage, la médiane des profondeurs de couverture de séquençage, le nombre de bases cartographiées sur les chromosomes individuels, la profondeur moyenne de couverture de séquençage des chromosomes individuels, l'écart-type des moyennes de profondeurs de couverture de séquençage des chromosomes individuels..

3. Procédé selon la revendication 2, dans lequel les métriques statistiques calculées sur les données de séquençage cartographiées de l'échantillon testé à l'étape c) comprennent les mêmes métriques statistiques que celles utilisées lors de la création de l'ensemble de données d'apprentissage.

4. Procédé selon l'une quelconque des revendications précédentes, dans lequel les statistiques proviennent de données génomiques incomplètes

5. Modèle statistique ou modèle d'apprentissage automatique pour déterminer la présence d'une tumeur à partir de métriques de cartographie de fragments d'ADN libre circulant dans un échantillon testé, utilisé dans le procédé selon l'une quelconque des revendications précédentes, comprenant en entrée des métriques statistiques issues des données de séquençage cartographiées de l'échantillon, fournissant, pour le nombre de lectures cartographiées, le nombre de lectures non cartographiées et la longueur des fragments d'ADN en entrée, une information sur la présence d'une tumeur en sortie.

6. Modèle selon la revendication 5, comprenant en outre en entrée n'importe quelle donnée parmi l'ensemble contenant : le nombre de lectures séquencées, le ratio des lectures cartographiées sur le génome de référence par rapport à toutes les lectures, le nombre de lectures avec différentes positions sur le génome au sein d'une même lecture, le ratio de toutes les lectures avec différentes positions sur le génome au sein d'une même lecture par rapport à toutes les lectures, le nombre de lectures avec deux positions ou plus sur le génome, le ratio de toutes les lectures avec deux positions ou plus sur le génome, le nombre de paires de lectures avec la première lecture de la paire cartographiée, le nombre de paires de lectures avec la deuxième lecture de la paire cartographiée, le nombre de paires de lectures avec les deux lectures de la paire cartographiées, le nombre de paires de lectures avec une seule lecture de la paire cartographiée, le nombre de bases séquencées, le nombre de bases cartographiées, le nombre de lectures marquées comme dupliquées, la qualité moyenne de cartographie, la médiane de la qualité de cartographie, le nombre de bases adénine dans les lectures cartographiées, le ratio des bases adénine par rapport à toutes les bases, le nombre de bases cytosine dans les lectures cartographiées, le ratio des bases cytosine par rapport à toutes les bases, le nombre de bases thymine dans les lectures cartographiées, le ratio des bases thymine par rapport à toutes les bases, le nombre de bases guanine dans les lectures cartographiées, le ratio des bases guanine par rapport à toutes les bases, le nombre de bases inconnues dans les lectures cartographiées, le ratio des bases inconnues par rapport à toutes les bases, le ratio de mésappariements de bases par rapport à toutes les bases cartographiées, le nombre de bases substituées par rapport à la référence, le nombre de bases insérées par rapport à la référence, le nombre de délétions par rapport à la référence, le nombre de lectures avec insertion, le nombre de lectures avec délétion, le nombre d'insertions et de délétions homopolymériques, la profondeur moyenne de couverture de séquençage, l'écart-type des profondeurs de couverture de séquençage, la médiane des profondeurs de couverture de séquençage, le nombre de bases cartographiées sur les chromosomes individuels, la profondeur moyenne de couverture de séquençage des chromosomes individuels, l'écart-type des moyennes de profondeurs de couverture de séquençage des chromosomes individuels.

7. Procédé mis en œuvre par ordinateur pour la création d'un ensemble de données d'apprentissage pour un modèle statistique ou un modèle d'apprentissage automatique selon la revendication 5, comprenant les étapes suivantes:
a. Création d'un ensemble de données d'apprentissage par le calcul de métriques statistiques sur des données de séquençage cartographiées provenant d'échantillons d'apprentissage, comprenant le nombre de lectures cartographiées, le nombre de lectures non cartographiées, la longueur des fragments d'ADN et l'information sur la présence d'une tumeur;
a. Entraînement d'un modèle statistique ou d'un modèle d'apprentissage automatique en utilisant l'information sur la présence d'une tumeur de l'étape a) comme sortie du modèle et les autres métriques statistiques de l'étape a) comme entrée du modèle.

8. Procédé selon la revendication 7, dans lequel les métriques statistiques calculées sur les données de séquençage cartographiées provenant des échantillons d'apprentissage à l'étape a) comprennent en outre n'importe quelle donnée parmi l'ensemble contenant : le nombre de lectures séquencées, le ratio des lectures cartographiées sur le génome de référence par rapport à toutes les lectures, le nombre de lectures avec différentes positions sur le génome au sein d'une même lecture, le ratio de toutes les lectures avec différentes positions sur le génome au sein d'une même lecture par rapport à toutes les lectures, le nombre de lectures avec deux positions ou plus sur le génome, le ratio de toutes les lectures avec deux positions ou plus sur le génome, le nombre de paires de lectures avec la première lecture de la paire cartographiée, le nombre de paires de lectures avec la deuxième lecture de la paire cartographiée, le nombre de paires de lectures avec les deux lectures de la paire cartographiées, le nombre de paires de lectures avec une seule lecture de la paire cartographiée, le nombre de bases séquencées, le nombre de bases cartographiées, le nombre de lectures marquées comme dupliquées, la qualité moyenne de cartographie, la médiane de la qualité de cartographie, le nombre de bases adénine dans les lectures cartographiées, le ratio des bases adénine par rapport à toutes les bases, le nombre de bases cytosine dans les lectures cartographiées, le ratio des bases cytosine par rapport à toutes les bases, le nombre de bases thymine dans les lectures cartographiées, le ratio des bases thymine par rapport à toutes les bases, le nombre de bases guanine dans les lectures cartographiées, le ratio des bases guanine par rapport à toutes les bases, le nombre de bases inconnues dans les lectures cartographiées, le ratio des bases inconnues par rapport à toutes les bases, le ratio de mésappariements de bases par rapport à toutes les bases cartographiées, le nombre de bases substituées par rapport à la référence, le nombre de bases insérées par rapport à la référence, le nombre de délétions par rapport à la référence, le nombre de lectures avec insertion, le nombre de lectures avec délétion, le nombre d'insertions et de délétions homopolymériques, la profondeur moyenne de couverture de séquençage, l'écart-type des profondeurs de couverture de séquençage, la médiane des profondeurs de couverture de séquençage, le nombre de bases cartographiées sur les chromosomes individuels, la profondeur moyenne de couverture de séquençage des chromosomes individuels, l'écart-type des moyennes de profondeurs de couverture de séquençage des chromosomes individuels.

9. Système informatique, comprenant des dispositifs de calcul qui sont configurés pour exécuter le procédé selon l'une quelconque des revendications 1 à 4, 7 ou 8.

10. Programme d'ordinateur comprenant des instructions qui, lorsqu'elles sont exécutées par un ordinateur, assurent la mise en œuvre du procédé selon l'une quelconque des revendications 1 à 4, 7 ou 8 par cet ordinateur

11. Support de données informatique comprenant des instructions de programme qui, lorsqu'elles sont exécutées par un ordinateur, assurent la mise en œuvre du procédé selon l'une quelconque des revendications 1 à 4, 7 ou 8 par cet ordinateur.
